# FASCICULE DE BREVET EUROPEEN

(11) **EP 0 882 135 B1**
(45) Date de publication et mention de la délivrance du brevet: **15.05.2002**
(21) Numéro de dépôt: 97906233.8
(22) Date de dépôt: 20.02.1997
(51) Int. Cl.: C12P 5/02, C12N 1/20, C02F 3/28, A23K 1/00

(54) **PROCEDE ET INSTALLATION DE TRAITEMENT DES DECHETS ORGANIQUES ET APPLICATIONS DUDIT PROCEDE**
VERFAHREN UND VORRICHTUNG ZUR BEHANDLUNG VON ORGANISCHEN ABFALLSTOFFEN UND ANWENDUNG DES VERFAHRENS
ORGANIC WASTE PROCESSING METHOD AND PLANT, AND USES OF SAID METHOD

(30) Priorité: 21.02.1996 FR 9602131
(43) Date de publication de la demande: 09.12.1998
(73) Titulaire: AGENCE SPATIALE EUROPEENNE, F-75738 Paris Cedex 15 (FR)
(72) Inventeur: LASSEUR, Christophe, André, Antoine, NL-2191 AC De Zilk (NL); RICHALET, Jacques, Ernest, Nicolas, F-78430 Louveciennes (FR); VERSTRAETE, Willy, Henry, B-9032 Wondelgem (BE)
(74) Mandataire: Vialle-Presles, Marie José
(86) Numéro de dépôt international: FR9700316
(87) Numéro de publication internationale: WO9731120

(56) Documents cités:
- WO-A-84/00038
- WO-A-93/04988
- FR-A- 2 477 522
- US-A- 3 930 998
- US-A- 4 277 342
- US-A- 4 919 813
- US-A- 5 250 427
- DATABASE WPI Section Ch, Week 7832 Derwent Publications Ltd., London, GB; Class C04, AN 78-57463A XP002019479 & JP 53 075 072 A (NAKAMURA M) , 4 juillet 1978
- DATABASE WPI Section Ch, Week 9317 Derwent Publications Ltd., London, GB; Class D13, AN 93-141626 XP002019941 & SU 1 731 809 A (AS ARMN MICRIBIOL INST) , 7 mai 1992
- DATABASE WPI Section Ch, Week 9320 Derwent Publications Ltd., London, GB; Class D15, AN 93-163777 XP002019948 & JP 05 096 294 A (EBARA INFILCO KK) , 20 avril 1993

## Description

La présente Invention se rapporte à un procédé de traitement des déchets organiques, à une installation propre à permettre la mise en oeuvre de ce procédé ainsi qu'aux applications de ce procédé.

Le mot "environnement" a pris ces dernières années une importance considérable. A des conditions esthétiques (décharges sauvages, pollution visuelle), sont venues s'ajouter celles concernant l'hygiène et la sécurité, puis celles concernant la protection de l'homme, de la nature et des équilibres écologiques.

Devant la masse croissante des déchets à éliminer, les réglementations en matière de protection de l'environnement d'un certain nombre de pays Européens visent à une diminution de la quantité de déchets mis en décharge sans aucune valorisation, les décharges devant être réservées dans l'avenir aux résidus ultimes convenablement rendus inertes. Ainsi, seuls les déchets des déchets pourront être mis en décharge au profit d'une valorisation sous toutes ses formes.

Il s'en suit une réelle nécessité de développer des technologies propres à permettre un recyclage optimal des déchets et ce, quelle que soit leur origine et leur nature (déchets ménagers, agricoles, industriels, ...). Ces technologies doivent respecter les règles économiques de toute production et aboutir à des produits présentant une réelle valeur d'usage et des débouchés importants à des coûts de production satisfaisants. A cet égard, il est souhaitable que le coût énergétique du recyclage soit le plus faible possible.

Les déchets organiques d'origine animale et, notamment, les déjections produites par les élevages intensifs d'animaux domestiques, s'ils sont susceptibles de se décomposer spontanément au contraire d'autres déchets (déchets minéraux, macromolécules organiques de synthèse) qui nécessitent impérativement d'être traités, posent en particulier de sérieux problèmes d'environnement en raison des volumes très importants qu'ils représentent, des nuisances qu'ils génèrent (odeurs nauséabondes par exemple) et du caractère polluant, à hautes concentrations, des composés azotés issus de leur décomposition.

Dans un autre domaine, les stations de traitement et d'épuration des eaux usées sont confrontées à des problèmes de stockage et d'élimination des boues produites au cours de l'épuration de ces eaux, dont le volume ne cesse d'augmenter au fil des années et pour lesquelles elles ne disposent d'aucune possibilité de valorisation.

Pour traiter les déchets organiques, il a été proposé de les incinérer. L'incinération présente toutefois de nombreux inconvénients : elle est coûteuse tant en investissements qu'en exploitation, elle conduit à l'émission de fumées et de gaz qui nécessitent d'être traités à leur tour avec le poids économique correspondant et les résidus d'incinération ont une faible valeur commerciale.

On connaît également des procédés dits de "compostage" qui font intervenir une dégradation anaérobie des déchets par des bactéries et qui aboutissent à la formation de composts qui sont utilisés par les agriculteurs comme fertilisants. Ces procédés ne sont toutefois pas satisfaisants car la durée du traitement des déchets est élevée (20 jours en moyenne sont nécessaires pour obtenir un compost) et les composts obtenus qui sont pauvres en substances nutritives (azote, phosphore) sont des fertilisants de qualité médiocre. En outre, le compostage s'accompagne d'une émission d'odeurs nauséabondes qui implique un traitement particulier des gaz émis ou un apport de produits visant à atténuer la production de ces gaz.

On connaît aussi des procédés de traitement biologique des déchets organiques mais qui visent en général à diminuer la charge en produits toxiques et, notamment, en azote de ces déchets en vue de leur réintroduction dans le circuit de l'anabolisme.

Dans la demande de brevet FR-A-2 477 522 on décrit un procédé de traitement biologique d'effluants du type lisier. En plus du méthane qui est produit on obtient une épuration complète du lisier permettant le rejet dans le milieu naturel. Le document US-A-5 250 427 propose un procédé pour convertir des déchets organiques en une biomatière plastique. La première étape de ce procédé est une gazéification thermique. La biomatière plastique est produite par des bactéries à partir du gaz obtenu lors de la première étape.

La Demanderesse s'est donc fixée pour but de fournir un procédé de traitement des déchets organiques, qu'il s'agisse de déchets d'origine animale, de boues issues de l'épuration des eaux ou autres, assurant une valorisation optimale de ces déchets, tant sur le plan quantitatif que qualitatif, en un faible nombre de jours, sans production de produits toxiques et dont l'exploitation soit de faible coût.

Par ailleurs, des procédés visant à résoudre le problème de la production, à bord des engins spatiaux, de nourriture, d'eau et d'oxygène à partir des déchets produits par les spationautes en vue de réduire les quantités de produits alimentaires, d'eau et d'oxygène à emporter au moment du départ et d'assurer une autonomie biologique de l'équipage, ont été proposés (MERGEAY et al., Proceedings of the 3rd Symposium on Space Thermal Control & Life Support System, Noordwijk, THE NETHERLANDS, 3-6 Oct. 1988). Ces procédés constituent un écosystème artificiel comportant quatre compartiments axéniques colonisés par des microorganismes dont les principales fonctions représentent les différentes étapes de l'écosystème (liquéfaction des déchets, nitrification, biosynthèse) ainsi qu'un cinquième compartiment dit compartiment "consommateur" et qui est représenté par l'équipage. Cet écosystème est conçu pour fonctionner en circuit fermé, dans des conditions anoxygéniques pour limiter la consommation d'oxygène, et avec une utilisation de tous les produits présents dans le circuit, sans possibilité de sortie de l'un d'entre eux à un moment quelconque. Il s'en suit qu'un tel écosystème ne peut s'appliquer au recyclage des déchets organiques sur terre en raison des contraintes techniques et économiques qu'il imposerait.

La Demanderesse a découvert que, pour résoudre un problème différent, à savoir celui de la valorisation des déchets organiques, des techniques proposées pour la réalisation d'un système de production de nourriture, d'eau et d'oxygène en circuit fermé, peuvent de manière surprenante être adaptées à la réalisation d'un système ouvert permettant de traiter à une échelle industrielle ces déchets et d'obtenir la production d'une part, d'une biomasse comestible à haute valeur nutritionnelle, utilisable pour la fabrication d'aliments pour animaux, d'autre part, de gaz combustibles aisément valorisables, et, enfin, de produits aptes à être utilisés comme fertilisants.

La présente Invention a donc pour objet un procédé de traitement en circuit ouvert des déchets organiques, caractérisé en ce qu'il comprend les étapes suivantes :
a) la collecte des déchets,
b) l'introduction des déchets sans stérilisation préalable dans un premier réacteur,
c) la dégradation des déchets, dans ledit premier réacteur, par des bactéries anaérobies mésophiles ou thermophiles,
d) le recueil de l'effluent liquide résultant de ladite dégradation et son transfert dans un deuxième réacteur contenant des bactéries hétérotrophes ou photohétérotrophes,
e) la production par les bactéries hétérotrophes ou photohétérotrophes d'une biomasse comestible constituée par lesdites bactéries,
f) le recueil de la biomasse ainsi produite et son conditionnement.
Au sens de la présente Invention, on entend :
   - par bactéries anaérobies mésophiles, des bactéries qui sont capables de se développer en l'absence d'oxygène et à des températures comprises entre 20 et 40°C ;
   - par bactéries anaérobies thermophiles, des bactéries qui sont capables de se développer en l'absence d'oxygène et à des températures supérieures à 45°C et pouvant atteindre 85°C ;
   - par bactéries hétérotrophes, des bactéries qui sont capables d'assimiler des sources carbonées organiques en utilisant l'oxygène comme source d'énergie et, partant, de se développer sur de telles sources ; et
   - par bactéries photohétérotrophes, des bactéries qui sont également capables de se développer sur des sources carbonées mais en utilisant la lumière comme source d'énergie.

Selon une première disposition préférée du procédé de traitement conforme à l'Invention, les bactéries anaérobies mésophiles ou thermophiles assurant la dégradation des déchets sont un mélange de bactéries protéolytiques, de bactéries saccharolytiques et de bactéries cellulolytiques, de manière à obtenir une hydrolyse simultanée des différents polymères biologiques (protéines, carbohydrates, ADN, ARN, lipides, cellulose, ...) entrant dans la constitution des déchets en petits composés carbonés et azotés aptes à être assimilés par les bactéries hétérotrophes ou photohétérotrophes présentes dans le deuxième réacteur.

Ces petits composés carbonés et azotés sont principalement des acides gras volatiles (acides acétique, acide valérique, butyrique, isobutyrique, propionique, caproïque, ...), des amines, de l'urée, de l'éthanol et de l'ammoniaque.

De manière avantageuse, les bactéries anaérobies mésophiles ou thermophiles assurant la dégradation des déchets sont des bactéries commensales des intestins des animaux.

Selon un mode de mise en oeuvre préféré du procédé de traitement conforme à l'Invention, la dégradation des déchets est réalisée par des bactéries anaérobies thermophiles à une température comprise entre 45 et 80°C et, de préférence, entre 55 et 70°C. L'utilisation d'une telle température permet, en effet, d'éliminer les germes pathogènes tels que bactéries, levures, protozoaires ou virus susceptibles d'être présents dans les déchets lors de leur introduction dans le premier réacteur, tout en permettant aux bactéries thermophiles chargées de la dégradation des déchets de se développer et de remplir leur fonction.

Conformément à l'Invention, la dégradation des déchets est réalisée pendant un temps suffisant pour obtenir des taux de dégradation des protéines, des polysaccharides et des lipides présents dans les déchets supérieurs à 80%. Cette dégradation se traduit par une liquéfaction des déchets et la production d'un effluent qui comprend une phase liquide et une phase solide formée de particules en suspension dans ladite phase liquide.

Selon une autre disposition préférée du procédé de traitement conforme à l'Invention, le recueil de l'effluent résultant de la dégradation des déchets comprend une étape de séparation des phases liquide et solide de cet effluent.

Cette séparation est réalisée, de préférence, par filtration ou par tamisage.

Selon encore une autre disposition préférée du procédé de traitement conforme à l'Invention, les bactéries hétérotrophes ou photohétérotrophes assurant la production de la biomasse comestible appartiennent à la famille des *Rhodospirillaceae*. En effet, ces bactéries sont capables de se développer sur un grand nombre de sources carbonées et, notamment, sur les acides gras volatiles présents dans l'effluent liquide issu de la dégradation des déchets, et ce, aussi bien en aérobiose, en l'absence de lumière (respiration) qu'en anaérobiose, en présence de lumière (photosynthèse), et permettent, de ce fait, d'obtenir la production de la biomasse comestible par croissance bactérienne, tant dans des conditions d'aérobiose que d'anaérobiose.

Parmi les *Rhodospirillaceae,* on utilise préférentiellement des bactéries du genre *Rhodobacter* telles que *Rhodobacter capsulatus* et/ou du genre *Rhodospirillum* telles que *Rhodospirillum rubrum.*

Conformément à l'Invention, la production de la biomasse comestible est avantageusement réalisée en hétérotrophie et aérobiose.

Selon encore une autre disposition préférée du procédé de traitement conforme à l'Invention, le recueil de la biomasse comestible comprend une étape de séparation de cette biomasse de l'effluent liquide dans lequel elle se trouve.

De préférence, cette séparation est réalisée par flottation.

Selon encore une autre disposition préférée du procédé de traitement conforme à l'Invention, celui-ci comprend, de plus, le prélèvement des gaz combustibles (CH₄, H₂, ...) produits au cours de la dégradation des déchets pour leur valorisation par combustion.

Avantageusement, la combustion des gaz est utilisée pour chauffer le premier réacteur.

Complémentairement, le procédé de traitement en circuit ouvert des déchets organiques conforme à l'Invention comprend le compostage des matières solides résiduelles de la dégradation desdits déchets pour leur valorisation sous la forme de composts.

Un tel procédé présente l'avantage, outre de résoudre les problèmes d'environnement posés par l'accumulation des déchets organiques, de conférer à ces derniers une valeur d'usage en permettant leur transformation en une biomasse comestible à haute valeur nutritionnelle apte à être utilisée dans l'alimentation animale, ainsi qu'en gaz aisément valorisables par combustion et en produits fertilisants et ce, sans production de résidus toxiques et à un coût compatible avec les exigences industrielles.

L'Invention a, également, pour objet une installation pour la mise en oeuvre du procédé de traitement en circuit ouvert des déchets organiques tel que défini ci-avant, caractérisée en ce qu'elle comprend :
a) un premier réacteur pour la dégradation des déchets par des bactéries anaérobies mésophiles ou thermophiles, comprenant une enceinte munie de moyens d'amenée des déchets et de moyens de recueil de l'efflùent liquide résultant de cette dégradation,
b) un deuxième réacteur pour la production par des bactéries hétérotrophes ou photohétérotrophes d'une biomasse comestible constituée par lesdites bactéries, ledit deuxième réacteur comprenant une enceinte munie de moyens de recueil de la biomasse produite,
c) des moyens pour transférer ledit effluent du premier réacteur dans le deuxième réacteur, et
d) des moyens de conditionnement de ladite biomasse.

Selon une première disposition préférée de l'installation conforme à l'Invention, l'enceinte du premier réacteur est munie de moyens de chauffage et/ou de moyens de prélèvement des gaz libérés dans ladite enceinte au cours de la dégradation des déchets.

De manière avantageuse, l'enceinte du premier réacteur est munie à la fois de moyens de chauffage et de moyens pour prélever les gaz libérés au cours de la dégradation des déchets, et les moyens de chauffage comprennent un système de chauffage alimenté par les moyens de prélèvement des gaz, directement ou par l'intermédiaire d'un dispositif de stockage.

Selon une autre disposition préférée de l'installation conforme à l'Invention, les moyens de recueil de l'effluent liquide résultant de la dégradation des déchets comprennent des moyens pour évacuer cet effluent de l'enceinte du premier réacteur et des moyens de séparation de phases pour séparer les phases liquide et solide qui le constituent.

Selon encore une autre disposition préférée de l'installation conforme à l'Invention, l'enceinte du deuxième réacteur est munie de moyens d'éclairage ou de moyens d'aération.

Selon un premier mode de réalisation avantageux de l'installation conforme à l'Invention, l'enceinte du deuxième réacteur est réalisée en partie ou totalement dans un matériau transparent et les moyens d'éclairage comprennent une source de lumière naturelle (lumière solaire) ou artificielle disposée à l'extérieur de ladite enceinte.

Selon un autre mode de réalisation avantageux de cette installation, les moyens d'éclairage de l'enceinte du deuxième réacteur comprennent une source de lumière artificielle disposée à l'intérieur de ladite enceinte.

Selon encore une autre disposition préférée de l'installation conforme à l'Invention, les moyens de recueil de la biomasse comestible comprennent des moyens pour évacuer cette biomasse de l'enceinte du deuxième réacteur et des moyens de séparation de phases pour la séparer de l'effluent liquide dans lequel elle se trouve.

Avantageusement, les moyens de conditionnement de la biomasse comestible comprennent des moyens pour l'égoutter et/ou des moyens pour la stériliser et/ou des moyens pour la déshydrater.

Selon un mode de réalisation particulièrement préféré de l'installation conforme à l'Invention, celle-ci comprend, de plus, une unité de compostage pour la valorisation des matières solides résiduelles de la dégradation des déchets sous la forme de composts.

La présente Invention a, aussi, pour objet l'application du procédé de traitement en circuit ouvert des déchets organiques tel que défini ci-avant au recyclage des déchets d'origine animale tels que par exemple, les déjections présentes dans les litières des animaux d'élevage (porcs, ruminants, équidés, volailles, ...), dans les lisiers ou les purins.

La présente Invention a, encore, pour objet l'application de ce procédé au recyclage des boues issues de l'épuration des eaux.

La présente Invention a, en outre, pour objet l'application de ce même procédé à la fabrication d'aliments pour animaux.

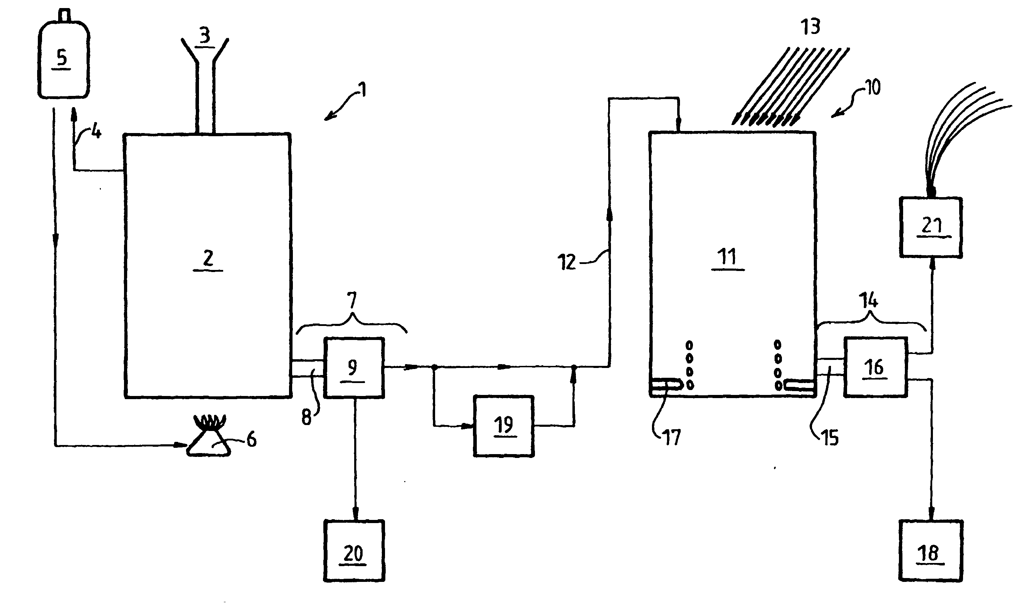

D'autres caractéristiques et avantages de l'Invention apparaîtront du complément de description qui suit et qui se réfère à la figure annexée qui représente un schéma de principe d'une installation conforme à l'Invention, ainsi qu'à des exemples d'application de l'Invention au traitement du lisier de porcs.

Il va de soi, toutefois, que ce complément de description est donné uniquement à titre d'illustration de l'objet de l'Invention et ne constitue en aucune manière une limitation de celui-ci.

On se réfère tout d'abord à la figure annexée.

Une installation conforme à l'Invention - qui peut être sur site de production des déchets ou hors site - comporte un premier réacteur (1) qui comprend une enceinte (2) propre à recevoir un volume déterminé de déchets pour leur dégradation par les bactéries anaérobies mésophiles ou thermophiles. L'enceinte (2) est munie de moyens (3) d'amenée des déchets à partir de leur zone de production ou d'une fosse de réception et de stockage, qui peuvent être du type gravitaires, hydrauliques, mécaniques ou autres.

L'enceinte (2) peut être une enceinte ouverte du type fosse ou bassin et être éventuellement équipée de moyens amovibles de fermeture. Elle peut également être une enceinte fermée du type cuve ou digesteur.

Selon la nature des déchets à traiter et les conditions dans lesquelles est réalisée leur dégradation (température, pH, temps de séjour des déchets dans l'enceinte, ...), celle-ci s'accompagne d'une production plus ou moins importante de gaz combustibles (CH₄, H₂, ...). Aussi, le premier réacteur (1) peut avantageusement comprendre des moyens (4) de prélèvement des gaz combustibles libérés dans l'enceinte (2) au cours de la dégradation des déchets pour leur valorisation par combustion, immédiate ou différée avec, dans ce dernier cas, un acheminement des gaz prélevés vers un dispositif (5) de stockage comprenant par exemple des compresseurs ou des liquéfacteurs et des bouteilles.

Conformément à l'Invention, la dégradation des déchets peut être mésophile ou thermophile, c'est-à-dire qu'elle peut être réalisée à des températures qui se situent entre 20 et 80°C. Selon la température choisie pour effectuer cette dégradation, il peut être nécessaire, voire avantageux de munir l'enceinte (2) du premier réacteur (1) de moyens de chauffage (6) permettant d'amener et de maintenir les déchets à cette température.

Ces moyens (6) de chauffage qui peuvent comprendre par exemple un ou plusieurs serpentins de circulation d'un fluide caloriporteur comme de l'eau chaude reliés à une source de chaleur (chaudière par exemple), peuvent être alimentés exclusivement par une source d'énergie externe à l'installation (chauffage électrique, chauffage au fuel, chauffage au gaz, ...).

Toutefois, lorsque l'enceinte (2) du premier réacteur (1) est également munie de moyens pour prélever les gaz libérés au cours de la dégradation des déchets, il peut être extrêmement avantageux d'utiliser ces gaz comme source d'énergie pour le chauffage de ladite enceinte.

Dans ce cas, les moyens (6) de chauffage de l'enceinte (2) comprennent par exemple un premier système de chauffage alimenté par une source d'énergie externe à l'installation et un deuxième système de chauffage indépendant du premier et comprenant un ou plusieurs brûleurs alimentés par les moyens (4) de prélèvement des gaz présents dans l'enceinte (2), directement ou par l'intermédiaire du dispositif (5) de stockage de ces gaz. En variante, les moyens (6) de chauffage de l'enceinte (2) comprennent un système de chauffage unique muni de deux entrées dont la première est reliée à une source d'énergie externe à l'installation tandis que la deuxième est alimentée par les moyens (4) de prélèvement des gaz de l'enceinte (2) et/ou le dispositif (5) de stockage de ceux-ci.

Avantageusement, l'enceinte (2) du premier réacteur (1) peut être isolée thermiquement et les moyens (6) de chauffage peuvent être pourvus d'un dispositif thermostatique permettant de maintenir les déchets à une température constante.

L'enceinte (2) du premier réacteur (1) peut également comprendre un dispositif de brassage, permanent ou intermittent, permettant d'assurer une homogénéité des déchets et de favoriser leur contact avec les bactéries.

Conformément à l'Invention, l'enceinte (2) du premier réacteur (1) est munie de moyens (7) de recueil de l'effluent liquide résultant de la dégradation des déchets.

Ces moyens (7) de recueil comprennent, d'une part, des moyens (8) pour évacuer l'effluent de l'enceinte (2) qui peuvent être constitués, par exemple, par un dispositif assurant une vidange partielle ou totale de cette enceinte (2), par pompage, syphonnage, écoulement ou autre. Ils comprennent, d'autre part, des moyens (9) de séparation de phases qui sont reliés auxdits moyens (8) d'évacuation et qui permettent de séparer les phases liquide et solide de l'effluent et, ainsi, de le débarrasser des matières solides en suspension qu'il renferme. Cette séparation de phases peut être réalisée par tout procédé classiquement utilisé pour séparer une phase liquide d'une phase solide : par filtration ou par tamisage au moyen d'un filtre ou d'un tamis muni d'ouvertures appropriées (idéalement, inférieures ou égales à 150 µm), par décantation dans un décanteur, par flottation dans un flottateur ou encore par centrifugation dans une centrifugeuse.

Comme également visible sur la figure annexée, l'installation conforme à l'Invention comporte un deuxième réacteur (10) qui comprend une enceinte (11) propre à recevoir, en provenance du premier réacteur (1) ou d'une cuve de stockage (19) intermédiaire, un volume déterminé d'effluent pour la production, par les bactéries hétérotrophes ou photohétérotrophes, de la biomasse comestible. Pour ce faire, l'installation conforme à l'Invention comprend des moyens de transfert (12) de l'effluent telle qu'une conduite d'écoulement dont les deux extrémités s'abouchent de façon appropriée d'une part, au premier réacteur (1) ou à la cuve de stockage (19) intermédiaire et, d'autre part, au deuxième réacteur (10).

Conformément à l'Invention, la production de la biomasse comestible est obtenue par croissance bactérienne, soit en hétérotrophie, soit en photohétérotrophie. La voie hétérotrophe nécessite de fournir de l'oxygène aux bactéries et implique, donc, des conditions d'aérobiose. La voie photohétérotrophe nécessite, elle, de leur fournir de la lumière et implique des conditions d'anaérobiose.

L'enceinte (11) du deuxième réacteur peut être prévue pour ne permettre l'utilisation que de l'une de ces deux voies de synthèse. Elle peut également avantageusement être conçue de manière à permettre la production de la biomasse comestible aussi bien en hétérotrophie et aérobiose qu'en photohétérotrophie et anaérobiose, en fonction des déchets à traiter, des critères climatiques, économiques ou autres. Ainsi, il peut s'agir d'une enceinte ouverte telle qu'un bassin, muni d'un système amovible permettant sa fermeture hermétique pour une utilisation en anaérobiose ou, au contraire, d'une enceinte hermétiquement close telle qu'une cuve, munie de moyens (17) d'aération pour une utilisation en aérobiose.

De tels moyens (17) d'aération comprennent avantageusement des moyens de type diffuseurs, placés en fond d'enceinte et permettant d'alimenter cette dernière en oxygène avec une pression suffisante pour vaincre la pression hydrostatique existant au niveau de ces diffuseurs.

Cette enceinte est, par ailleurs, munie de moyens (13) d'éclairage propres à fournir de la lumière aux bactéries lorsque la production de la biomasse comestible est réalisée en photohétérotrophie. L'enceinte peut alors être réalisée en partie ou en totalité dans un matériau transparent tel que du verre et être équipée d'un système d'éclairage externe composé d'un ensemble de lampes réparties de façon appropriée sur sa surface externe. En variante, l'enceinte (11), également réalisée en partie ou en totalité dans un matériau transparent, peut être éclairée par la lumière solaire directement ou par l'intermédiaire de miroirs propres à capter et à renvoyer sur sa surface externe les rayons du soleil. Dans encore une autre variante, un système d'éclairage artificiel de l'enceinte (11) peut être prévu à l'intérieur de celle-ci et non à l'extérieur. Bien entendu, il est également possible de prévoir un système d'éclairage utilisant, de manière simultanée ou alternée, deux sources de lumière, l'une naturelle et l'autre artificielle. Dans tous les cas, il peut être avantageux de munir l'enceinte (11) d'un dispositif permettant de régler l'intensité lumineuse fournie aux bactéries.

De plus, pour compenser la chaleur dégagée en condition opératoire par le ou les systèmes d'éclairage et maintenir la température interne de l'enceinte (11) à une valeur constante, celle-ci peut avantageusement présenter un système d'isolation thermique, voire de refroidissement du type circulation dans un échangeur de chaleur d'un fluide réfrigérant.

L'enceinte (11) du deuxième réacteur (10) peut comprendre, en outre, un dispositif d'agitation ou de brassage, intermittent ou continu.

Conformément à l'Invention, l'enceinte (11) du deuxième réacteur (10) est équipée de moyens (14) de recueil de la biomasse produite dans ladite enceinte. Ces moyens (14) de recueil comprennent, d'une part, des moyens pour évacuer tout ou partie du contenu de l'enceinte (11) hors de celle-ci et, d'autre part, des moyens pour séparer l'effluent liquide dans lequel elle baigne. Cette séparation peut être réalisée par filtration, par exemple, sur une membrane d'ultra-filtration, par décantation au moyen d'un décanteur lamellaire ou encore par flottation dans un flottateur.

Le deuxième réacteur (10) comprend des moyens (18) pour conditionner la biomasse. Par conditionnement, on entend toute opération ayant pour but de mettre la biomasse sous une forme désirée (blocs, pains, morceaux, pâtes, liquides, ...) et/ou sous une présentation appropriée à son transport (mise en containers par exemple) ou à sa commercialisation. Ainsi, ces moyens de conditionnement (18) peuvent comprendre des moyens pour égoutter la biomasse et, ainsi, la concentrer, des moyens pour la stériliser et des moyens pour la déshydrater de manière à la valoriser sous la forme d'un produit sec.

Comme également visible sur la figure annexée, l'installation conforme à l'Invention comprend une unité de compostage (20) pour valoriser les matières solides résiduelles de la dégradation des déchets sous la forme de composts, ainsi qu'un dispositif (21) pour le stockage de l'effluent résiduel en vue de son épandage ultérieur sur des terres agricoles.

Le compostage des matières solides peut être réalisé dans toute installation classiquement utilisée pour le compostage des déchets. Selon l'Invention, on préfère toutefois soumettre les matières solides à un compostage accéléré sous aération forcée qui, en raison de ce qu'il constitue un processus biologique thermophile, permet de les déshydrater, de les stabiliser (suppression des mauvaises odeurs) et de détruire les germes pathogènes qu'elles sont susceptibles de contenir. Pour ce faire, l'unité de compostage (20) comprend un ou plusieurs silos qui sont équipés d'un ou plusieurs systèmes de ventilation propres à assurer en permanence leur aération.

L'installation conforme à l'Invention peut comporter un système de contrôle et de commande comprenant une unité centrale du type ordinateur ou automate programmable recevant, par l'intermédiaire de sondes et/ou de capteurs répartis dans le dispositif de manière appropriée, les informations relatives aux différents paramètres (température, pH, intensité lumineuse, pression, vitesse d'écoulement, état d'ouverture ou de fermeture des vannes, ...) et comportant des sorties connectées à divers régulateurs propres à corriger les valeurs de ces paramètres si nécessaire et à optimiser les conditions de mise en oeuvre du procédé de traitement des déchets.

L'installation conforme à l'Invention peut être conçue de manière à assurer un traitement des déchets par lots, en semi-continu et/ou en continu.

Pour la mise en oeuvre du procédé conforme à l'Invention, les déchets à traiter sont introduits dans l'enceinte (2) du premier réacteur (1) pour y être dégradés. Cette dégradation est avantageusement réalisée par une population de bactéries anaérobies associant des bactéries protéolytiques, des bactéries saccharolytiques et des bactéries cellulolytiques, de manière à obtenir une hydrolyse simultanée des différents polymères (protéines, carbohydrates, ADN, ARN, lipides, cellulose, ...) présents dans les déchets.

Les bactéries commensales des intestins des animaux constituent de telles populations. Elles sont, en outre, généralement capables de supporter des températures supérieures à 45°C. De ce fait, dans le cas de déchets constitués par des déjections animales telles que les lisiers ou les purins, la dégradation des déchets, qu'elle soit mésophile ou thermophile, est avantageusement assurée par les bactéries naturellement présentes dans ces déchets.

Dans le cas d'autres déchets, il peut être nécessaire, voire avantageux d'introduire dans l'enceinte (2) du premier réacteur (1), conjointement avec lesdits déchets, une population de souches bactériennes judicieusement choisies (*Clostridium thermocellum, Clostridium thermosaccharolyticum, Coprothermobacter prokeolyticus, Bacteroides, Bifidobacterium, Lactobacillus, Escherichia coli, Eubacterium, Peptococcus, Enterobacter*) et dont certaines peuvent être connues pour être commensales des intestins des animaux.

Conformément à l'Invention, la dégradation des déchets peut être réalisée à des températures qui se situent entre 20 et 80°C. Dans la mesure où les déchets sont introduits dans l'enceinte (2) du premier réacteur (1) sans être soumis préalablement à une stérilisation, il peut être extrêmement avantageux d'effectuer leur dégradation à une température comprise entre 45 et 80°C et, de préférence, entre 50 et 70°C, de manière à éliminer les germes pathogènes qu'ils sont susceptibles de contenir.

Dans tous les cas, la dégradation des déchets est conduite pendant un temps suffisant pour obtenir des taux de dégradation des protéines, des polysaccharides et des lipides présents dans ces déchets au moins égaux à 80% et, préférentiellement, supérieurs à 85%. Ce temps varie en fonction de la composition des déchets et des conditions dans lesquelles leur dégradation est réalisée (température, souches bactériennes utilisées, ...). Il est donc possible et même souhaitable de déterminer, pour chaque type de déchets à traiter, le temps de séjour optimal de ces déchets dans l'enceinte (2) du premier réacteur (1) en faisant varier les conditions de déroulement de leur dégradation.

La dégradation des déchets se traduit par une liquéfaction de ces déchets et leur transformation en un effluent liquide composé de deux phases : l'une liquide, qui renferme des acides gras volatiles (acides acétique, acide valérique, butyrique, isobutyrique, propionique, caproique, ...), des amines, de l'urée, de l'éthanol, de l'ammoniaque, ..., et l'autre solide, formée par des particules en suspension de matières non dégradées.

La dégradation des déchets se traduit, par ailleurs, par une production de gaz combustibles et, plus particulièrement de méthane, dont l'importance dépend d'un certain nombre de paramètres et, notamment, de la température à laquelle est conduite la dégradation des déchets, leur pH, leur concentration en ammoniaque et leur temps de séjour dans l'enceinte (2) du premier réacteur (1), comme c'est classiquement le cas dans les fermentations méthaniques. Il est donc possible de favoriser ou, au contraire, de limiter la production de ces gaz, en jouant sur ces paramètres.

Une fois évacué de l'enceinte (2) du premier réacteur (1), l'effluent liquide résultant de la dégradation des déchets est avantageusement soumis à une séparation de phases de manière à épurer sa phase liquide des matières solides en suspension qu'elle renferme. Cette phase liquide est alors transférée dans l'enceinte (11) du deuxième réacteur (10), pour servir de substrat aux bactéries hétérotrophes ou photohétérotrophes et permettre, ainsi, la production de la biomasse.

Conformément à l'Invention, ces bactéries sont avantageusement choisies parmi les bactéries appartenant à la famille des *Rhodospirillaceae* et, plus particulièrement, aux genres *Rhodobacter* et *Rhodospirillum*, en raison de leur capacité à assimiler, aussi bien en hétérotrophie qu'en photohétérotrophie, un grand nombre de sources carbonées et notamment les acides gras volatiles présents dans la phase liquide de l'effluent.

La production de la biomasse est conduite à une température comprise entre environ 20 et 30°C et à un pH proche de la normalité. Son rendement est fonction du taux de croissance propre aux bactéries utilisées et, pour une souche bactérienne donnée, des conditions dans lesquelles elle est réalisée et, notamment, de l'utilisation ou non de lumière.

Les bactéries, par leur croissance, fournissent une biomasse qui se compose de protéines, de carbohydrates, de lipides et d'acides nucléiques et qui, une fois séparée de l'effluent résiduel dans lequel elle baigne et stérilisée, est apte à être utilisée dans l'alimentation animale.

Les matières solides résiduelles de la dégradation qui, outre de contenir de la matière organique non dégradée, sont riches en phosphore et en azote organique, sont, elles, acheminées jusqu'à l'unité de compostage (20) pour y être valorisées sous la forme de composts.

### EXEMPLES : TRAITEMENT DU LISIER DANS UN ELEVAGE PORCIN

La faisabilité industrielle du procédé et de l'installation de traitement des déchets conformes à l'Invention a été vérifiée dans leurs applications à l'épuration du lisier sur un site d'élevage intensif de porcs, par extrapolation des résultats expérimentaux obtenus sur des volumes réduits de déchets et par simulation du procédé et de l'installation à une échelle industrielle.

Cette vérification a été réalisée en tenant compte des contraintes d'exploitation (temps de main d'oeuvre, espace disponible pour le traitement du lisier, ...), de coûts et d'hygiène spécifiques à un tel élevage.

L'élevage considéré est un élevage de type "Naisseurs-Engraisseurs" comprenant 150 truies naisseuses donnant naissance à environ 2 700 porcs par an (soit une moyenne de 18 porcs par truie).

Deux modes différents de mise en oeuvre du procédé (ci-après "Filière 1" et "Filière 2") utilisant deux variantes de l'installation ont été validés sur la base d'une production journalière moyenne de lisier de 10,1 m³, dans les conditions et avec les résultats suivants.

### 1) Filière 1 :

La Filière 1 réalise :
- une dégradation mésophile du lisier à une température de 30°C,
- une filtration de l'effluent résultant de cette dégradation et un compostage des matières solides recueillies au cours de cette filtration,
- une production de biomasse comestible en photohétérotrophie et anaérobiose, et
- un épandage de l'effluent résiduel.

### a) la dégradation du lisier :

La dégradation du lisier est réalisée dans une fosse située sous le bâtiment d'élevage des porcs (par exemple, en dessous des caillebotis) et qui est alimentée en lisier par des moyens gravitaires. Prévue pour présenter une capacité correspondant à 2 mois de production de lisier, cette fosse présente un volume utile minimum de 650 m³.

La dégradation du lisier est effectuée par les bactéries naturellement présentes dans celui-ci, à une température de 30°C, laquelle, compte-tenu de ce que la température des bâtiments d'élevage est généralement maintenue à 20°C, est obtenue par un dispositif de chauffage tel qu'un serpentin alimenté par une circulation d'eau chaude et immergé dans le lisier. La dégradation du lisier est, par ailleurs, réalisée à un pH compris entre 5,9 et 6,8 de manière à limiter la production de méthane, ce qui nécessite de procéder à une légère acidification du lisier dont le pH se situe entre 7 et 7,6.

Dans ces conditions, on obtient en 14 jours des taux de dégradation des protéines, des polysaccharides et des lipides initialement présents dans le lisier supérieurs à 85%.

L'effluent résultant de cette dégradation est recueilli par des vidanges de la fosse, par exemple par des chasses ponctuelles dont le volume et la fréquence (toutes les deux semaines, tous les mois, voire tous les deux mois) sont réglés sur ceux de l'alimentation de la fosse en lisier. L'effluent est ensuite amené, par exemple par l'intermédiaire d'une conduite d'écoulement, jusqu'à une unité de filtration.

### b) la filtration de l'effluent :

La filtration de l'effluent est réalisée au moyen d'un filtre rotatif qui mesure 0,7 m de longueur, 0,5 m de largeur et 0,7 m de hauteur et qui est muni d'ouvertures de 150 µm. Ce filtre est alimenté en effluent par une pompe centrifuge à roue ouverte d'un débit de 1 m³/heure et il est entraîné en rotation par un moto-réducteur à vitesse variable. Il est, en outre équipé, d'un système de lavage sous pression permettant d'éviter son encrassement par les matières solides présentes dans l'effluent.

Cette filtration permet d'éliminer au moins 60% des matières solides qui se trouvent en suspension dans l'effluent, et conduit à la production d'un refus de filtration d'un volume de 1,6 m³/jour et comprenant 20% en poids de matières sèches (soit une production de 320 kg de matières sèches par jour), laissant ainsi un volume d'effluent filtré de 8,5 m³/jour.

L'effluent filtré est alors transféré dans le compartiment prévu pour la production de la biomasse comestible, par exemple au moyen d'une conduite d'écoulement, soit directement, soit après un stockage transitoire dans une cuve intermédiaire.

### c) la production de la biomasse comestible :

La biomasse comestible est produite par une culture de *Rhodobacter capsulatus*, qui est réalisée en photohétérotrophie et en anaérobiose dans une cuve conçue pour un temps de séjour de l'effluent de 7 jours et présentant, de ce fait, un volume utile de 66 m³.

Cette cuve présente une surface de 22 m² et une hauteur utile de 3 m. Elle est munie de moyens d'éclairage artificiel comprenant 120 néons de 150 W répartis en 5 niveaux de 24 néons chacun et assurant un éclairage permanent d'une puissance totale d'éclairage de 18 KW. La cuve est calorifugée et comprend, de plus, un dispositif d'agitation permanente.

La température de la culture est de 30°C et son pH est de 6,9.

Dans ces conditions, on obtient une production de biomasse d'environ 0,08 g de matière sèche par litre et par heure, soit une production de biomasse d'environ 160 kg de matière sèche par jour.

La biomasse ainsi produite est séparée de l'effluent par flottation au moyen d'un flottateur situé en aval de la cuve et alimenté par cette dernière, par exemple, par l'intermédiaire d'une conduite d'écoulement. Le flottateur permet de séparer les matières solides présentes en suspension dans la phase liquide (effluent) par l'injection d'un mélange d'air et d'eau pressurisé à 5-6 bars. Les bulles générées par ce mélange s'accrochent auxdites matières et les ramènent à la surface de la phase liquide où elles peuvent alors être récupérées par un raclage.

On utilise un flottateur dont les, dimensions permettent de traiter 1 m³/heure du mélange de biomasse et d'effluent.

La biomasse recueillie à l'issue de la flottation présente une concentration comprise entre 30 et 60 g de matière sèche par litre. Il est possible de la concentrer davantage en la laissant égoutter plusieurs jours dans une benne d'égouttage, puis de la soumettre à une déshydratation si l'on désire la valoriser sous la forme d'un produit sec.

Cette biomasse a une concentration en protéines qui représente environ 50% en poids de son poids sec et une valeur nutritive au moins équivalente à celle du soja.

### d) le compostage des matières solides recueillies au cours de la filtration :

Le refus de filtration est soumis à un compostage accéléré en silos sous aération forcée. Le volume du refus de filtration à composter étant de 1,6 m³/jour (soit 320 kg de matières sèches par jour), ce compostage est réalisé au moyen de deux silos couloirs identiques d'une capacité utile de 30 m³ (hauteur utile : 2 m, largeur utile : 3 m, longueur utile : 5 m) et qui sont aérés chacun par un ventilateur présentant un débit de 220 m³/heure et un différentiel de pression de 200 mm de colonne d'eau.

Le temps de séjour du refus de filtration dans les silos est de 60 jours.

On obtient ainsi la production d'un volume de compost de 0,45 m³/jour correspondant à une masse de compost d'environ 460 kg/jour. Ce compost présente des teneurs en matières organiques, en azote et en phosphore qui sont respectivement égales à 56%, 2,2% et 3,7% en poids, et peut avantageusement être utilisé comme support de culture.

### e) l'épandage de l'effluent résiduel :

La Filière 1 permet de réduire les charges initiales en azote et en phosphore du lisier respectivement de 68% et de 69% et de réduire sa DCO initiale de 91%.

De ce fait, l'effluent résiduel peut être épandu, après une période réglementaire de stockage d'au moins 4 mois, sur des terres agricoles pour servir de fertilisant.

### 2) Filière 2 :

La Filière 2 réalise :
- une dégradation mésophile du lisier à une température de 30°C,
- une filtration de l'effluent résultant de cette dégradation et le compostage des matières solides recueillies au cours de cette filtration,
- une production de biomasse comestible en hétérotrophie et en aérobiose, et
- un épandage de l'effluent résiduel.

La dégradation mésophile du lisier, la filtration de l'effluent résultant de cette dégradation, ainsi que le compostage des matières solides recueillies au cours de cette filtration sont réalisés dans les mêmes conditions que celles utilisées dans la Filière 1.

Par contre, la biomasse comestible est produite par une culture de *Rhodobacter capsulatus*, qui est réalisée en hétérotrophie et en aérobiose dans une cuve présentant un volume utile de 70 m³, soit une surface de 14 m² pour une hauteur utile de 5 m.

Cette cuve est munie d'un système d'aération comprenant un surpresseur d'un débit de 130 m³/heure relié à une réserve d'oxygène, ainsi que des diffuseurs situés en fond de cuve et permettant de fournir à la culture de *Rhodobacter capsulatus* une quantité journalière d'oxygène de 117 kg.

Le temps de séjour de l'effluent dans la cuve est de 7 jours. La température de la culture est de 30°C et son pH est de 6,9.

Dans ces conditions, on obtient une production de biomasse d'environ 0,05 g de matière sèche par litre et par heure, soit une production de biomasse d'environ 100 kg de matière sèche par jour.

Comme dans la Filière 1, la biomasse est séparée de l'effluent par flottation au moyen d'un flottateur situé en aval de la cuve et alimenté par cette dernière. La biomasse recueillie à l'issue de la flottation présente une concentration comprise entre 30 et 60 g de matière sèche par litre.

La Filière 2 permet de réduire les charges initiales en azote et en phosphore du lisier respectivement de 48% et de 69% et de réduire sa DCO initiale de 91%. Là encore, l'effluent résiduel peut être épandu, après une période réglementaire de stockage d'au moins 4 mois, sur des terres agricoles pour servir de fertilisant.

Le Tableau 2 ci-après présente les principales performances des Filières 1 et 2.

**Tableau 2**

| | **PRODUCTION DE BIOMASSE en kg de matière sèche/m**^{**3**} | **PRODUCTION DE COMPOST en kg de matière sèche/m**^{**3**} | **PERFORMANCES EPURATOIRES** | | |
|---|---|---|---|---|---|
| | | | ΔN | ΔP | ΔDCO |
| **FILIERE 1** | 16 | 34 | 68% | 69% | 91% |
| **FILIERE 2** | 10 | 34 | 48% | 69% | 91% |

## Revendications

1. Procédé de traitement en circuit ouvert des déchets organiques, **caractérisé en ce qu'**il comprend les étapes suivantes :
a) la collecte des déchets,
b) l'introduction des déchets sans stérilisation préalable dans un premier réacteur,
c) la dégradation des déchets, dans ledit premier réacteur, par des bactéries anaérobies mésophiles ou thermophiles,
d) le recueil de l'effluent liquide résultant de ladite dégradation et son transfert dans un deuxième réacteur contenant des bactéries hétérotrophes ou photohétérotrophes,
e) la production par les bactéries hétérotrophes ou photohétérotrophes d'une biomasse comestible constituée par lesdites bactéries,
f) le recueil de la biomasse ainsi produite et son conditionnement.

2. Procédé selon la Revendication 1, **caractérisé en ce que** les bactéries anaérobies mésophiles ou thermophiles assurant la dégradation des déchets sont un mélange de bactéries protéolytiques, de bactéries saccharolytiques et de bactéries cellulolytiques.

3. Procédé selon la Revendication 1 ou la Revendication 2, **caractérisé en ce que** les bactéries anaérobies mésophiles ou thermophiles assurant la dégradation des déchets sont des bactéries commensales des intestins des animaux.

4. Procédé selon l'une quelconque des Revendications précédentes, **caractérisé en ce que** la dégradation des déchets est réalisée par des bactéries anaérobies thermophiles à une température comprise entre 45 et 80°C et, de préférence, entre 55 et 70°C.

5. Procédé selon l'une quelconque des Revendications précédentes, **caractérisé en ce que** la dégradation des déchets est réalisée pendant un temps suffisant pour obtenir des taux de dégradation des protéines, des polysaccharides et des lipides présents dans les déchets supérieurs à 80%.

6. Procédé selon l'une quelconque des Revendications précédentes, **caractérisé en ce que** le recueil de l'effluent résultant de la dégradation des déchets comprend une étape de séparation des phases liquide et solide.

7. Procédé selon la Revendication 6, **caractérisé en ce que** la séparation des phases liquide et solide de l'effluent est réalisée par filtration ou par tamisage.

8. Procédé selon l'une quelconque des Revendications précédentes, **caractérisé en ce que** les bactéries hétérotrophes ou photohétérotrophes assurant la production de la biomasse comestible appartiennent à la famille des *Rhodospirillaceae.*

9. Procédé selon la Revendication 8, **caractérisé en ce que** les bactéries hétérotrophes ou photohétérotrophes assurant la production de la biomasse comestible appartiennent au genre *Rhodobacter* et/ou au genre *Rhodospirillum.*

10. Procédé selon l'une quelconque des Revendications précédentes, **caractérisé en ce que** la production de la biomasse comestible est réalisée en hétérotrophie et aérobiose.

11. Procédé selon l'une quelconque des Revendications précédentes, **caractérisé en ce que** le recueil de la biomasse comestible comprend une étape de séparation de cette biomasse de l'effluent liquide dans lequel elle se trouve.

12. Procédé selon la Revendication 11, **caractérisé en ce que** la séparation de la biomasse de l'effluent liquide est réalisée par flottation.

13. Procédé selon l'une quelconque des Revendications précédentes, **caractérisé en ce qu'**il comprend le prélèvement des gaz combustibles produits au cours de la dégradation des déchets par les bactéries anaérobies mésophiles ou thermophiles pour leur valorisation par combustion.

14. Procédé selon la Revendication 13, **caractérisé en ce que** la combustion des gaz est utilisée pour chauffer le premier réacteur.

15. Procédé selon l'une quelconque des Revendications précédentes, **caractérisé en ce qu'**il comprend le compostage des matières solides résiduelles de la dégradation des déchets pour leur valorisation sous la forme de composts.

16. Installation pour la mise en oeuvre d'un procédé de traitement en circuit ouvert des déchets organiques selon l'une quelconque des Revendications 1 à 11, **caractérisée en ce qu'**elle comprend :
a) un premier réacteur (1) pour la dégradation des déchets par des bactéries anaérobies mésophiles ou thermophiles, ledit réacteur comprenant une enceinte (2) munie de moyens (3) d'amenée des déchets et de moyens (7) de recueil de l'effluent liquide résultant de cette dégradation,
b) un deuxième réacteur (10) pour la production par des bactéries hétérotrophes ou photohétérotrophes d'une biomasse comestible constituée par lesdites bactéries, ledit deuxième réacteur (10) comprenant une enceinte (11) munie de moyens (14) de recueil de la biomasse produite,
c) des moyens (12) pour transférer l'effluent résultant de la dégradation des déchets du premier réacteur dans le deuxième réacteur, et
d) des moyens (18) de conditionnement de la biomasse.

17. Installation selon la revendication 16, **caractérisée en ce que** l'enceinte (2) du premier réacteur(1) est munie de moyens (6) de chauffage et/ou de moyens (4) de prélèvement des gaz libérés dans ladite enceinte au cours de la dégradation des déchets.

18. Installation selon la revendication 16 ou la revendication 17, **caractérisée en ce que** l'enceinte (2) du premier réacteur (1) est munie de moyens (6) de chauffage et de moyens (4) de prélèvement des gaz libérés au cours de la dégradation des déchets, et **en ce que** les moyens de chauffage comprennent un système de chauffage qui est alimenté par les moyens de prélèvement des gaz de ladite enceinte, directement ou par l'intermédiaire d'un dispositif (5) de stockage de ces gaz.

19. Installation selon l'une quelconque des revendications 16 à 18, **caractérisée en ce que** les moyens (7) de recueil de l'effluent liquide résultant de la dégradation des déchets comprennent des moyens (8) pour évacuer cet effluent de l'enceinte du premier réacteur et des moyens (9) de séparation de phases pour séparer les phases liquide et solide qui le constituent.

20. Installation selon l'une quelconque des revendications 16 à 20, **caractérisée en ce que** l'enceinte (11) du deuxième réacteur (10) est munie de moyens (13) d'éclairage ou de moyens (17) d'aération.

21. Installation selon la revendication 20, **caractérisée en ce que** l'enceinte (11) du deuxième. réacteur (10) est réalisée en partie ou totalement dans un matériau transparent et les moyens (13) d'éclairage comprennent une source de lumière naturelle (lumière solaire) ou artificielle disposée à l'extérieur de ladite enceinte.

22. Installation selon la revendication 20, **caractérisée en ce que** les moyens (13) d'éclairage de l'enceinte (11) du deuxième réacteur (10) comprennent une source de lumière artificielle disposée à l'intérieur de ladite enceinte.

23. Installation selon l'une quelconque des revendications 16 à 22, **caractérisée en ce que** les moyens (14) de recueil de la biomasse comestible comprennent des moyens (15) pour évacuer cette biomasse de l'enceinte du deuxième réacteur et des moyens (16) de séparation de phases pour la séparer de l'effluent liquide dans lequel elle se trouve.

24. Installation selon l'une quelconque des revendications 16 à 23, **caractérisée en ce que** les moyens (18) de conditionnement de la biomasse comestible comprennent des moyens pour égoutter et/ou des moyens pour stériliser et/ou des moyens pour déshydrater ladite biomasse.

25. Installation selon l'une quelconque des revendications 16 à 24, **caractérisée en ce qu'**elle comprend une unité de compostage (20) pour la valorisation des matières solides résiduelles de la dégradation des déchets sous la forme de composts.

26. Application du procédé de traitement en circuit ouvert des déchets organiques selon l'une quelconque des Revendications 1 à 15 au recyclage des déchets d'origine animale.

27. Application du procédé de traitement en circuit ouvert des déchets organiques selon l'une quelconque des Revendications 1 à 15 au recyclage des boues issues de l'épuration des eaux.

28. Application du procédé de traitement en circuit ouvert des déchets organiques selon l'une quelconque des Revendications 1 à 15 à la fabrication d'aliments pour animaux.

## Claims

1. An open circuit organic waste treatment method, **characterized in that** it comprises the following steps:
a) collecting the waste;
b) introducing the waste into a first reactor without prior sterilisation;
c) decomposing the waste in said first reactor using mesophilic or thermophilic anaerobic bacteria;
d) recovering the liquid effluent resulting from said decomposition and transferring it to a second reactor containing heterotrophic or photoheterotrophic bacteria;
e) using the heterotrophic or photoheterotrophic bacteria to produce an edible biomass constituted by said bacteria; and
f) recovering and packaging the biomass produced.

2. A method according to claim 1, **characterized in that** the mesophilic or thermophilic anaerobic bacteria which decompose the waste are a mixture of proteolytic, saccharolytic and cellulolytic bacteria.

3. A method according to claim 1 or claim 2, **characterized in that** the mesophilic or thermophilic anaerobic bacteria which decompose the waste are commensal animal intestine bacteria.

4. A method according to any one of the preceding claims, **characterized in that** waste decomposition is carried out using thermophilic anaerobic bacteria at a temperature in the range 45°C to 80°C, and preferably in the range 55°C to 70°C.

5. A method according to any one of the preceding claims, **characterized in that** waste decomposition is carried out for a time which is sufficient for the proteins, polysaccharides, and lipids present in the waste to be decomposed to a degree of over 80%.

6. A method according to any one of the preceding claims, **characterized in that** recovery of the effluent resulting from waste decomposition comprises a step for separating the liquid and solid phases of said effluent.

7. A method according to claim 6, **characterized in that** the liquid and solid phases of the effluent are separated by filtration or by screening.

8. A method according to any one of the preceding claims, **characterized in that** the heterotrophic or photoheterotrophic bacteria ensuring production of the edible biomass is from the *Rhodospirillaceae* family.

9. A method according to claim 8, **characterized in that** the heterotrophic or photoheterotrophic bacteria ensuring production of the edible biomass are from the genus *Rhodobacter* and/or from the genus *Rhodospirillum.*

10. A method according to any preceding claim, **characterized in that** the edible biomass is produced by heterotrophy and aerobiosis.

11. A method according to any preceding claim, **characterized in that** recovering the edible biomass comprises a step for separating this biomass from the liquid effluent in which it is immersed.

12. A method according to claim 11, **characterized in that** the biomass and liquid effluent are separated by flotation.

13. A method according to any one of the preceding claims, **characterized in that** it comprises removing the fuel gas produced during waste decomposition by mesophilic or thermophilic anaerobic bacteria to exploit it by combustion.

14. A method according to claim 13, **characterized in that** the gas combustion is used to heat the first reactor.

15. A method according to any one of the preceding claims, **characterized in that** it comprises composting the residual solid matter from waste decomposition to exploit it in the form of compost.

16. A plant for carrying out an open circuit organic waste treatment method according to any one of claims 1 to 11, **characterized in that** it comprises:
a) a first reactor (1) for decomposing waste using mesophilic or thermophilic anaerobic bacteria, said reactor comprising a vessel (2) provided with means (3) for supplying the waste and means (7) for recovering liquid effluent resulting from that decomposition;
b) a second reactor (10) using heterotrophic or photoheterotrophic bacteria to produce an edible biomass constituted by said bacteria, said second reactor (10) comprising a vessel (11) provided with means (14) for recovering the biomass produced;
c) means (12) for transferring the effluent resulting from waste decomposition from the first reactor to the second reactor; and
d) means (18) for packaging said biomass.

17. A plant according to claim 16, **characterized in that** the vessel (2) of the first reactor (1) is provided with means (6) for heating and/or means (4) for removing the gas released in said vessel during waste decomposition.

18. A plant according to claim 16 or claim 17, **characterized in that** the vessel (2) for the first reactor (1) is provided both with heating means (6) and with means (4) for removing the gas released during waste decomposition, and **in that** the heating means comprises a heating system supplied by the means for removing gas from said vessel, directly or via a means (5) for storing said gas.

19. A plant according to any one of claims 16 to 18, **characterized in that** the means (7) for recovering the liquid effluent resulting from waste decomposition comprises means (8) for evacuating the effluent from the vessel of the first reactor and means (9) for separating the phases to separate its liquid and solid phase constituents.

20. A plant according to any one of claims 16 to 20, **characterized in that** the vessel (11) of the second reactor (10) is provided with lighting means (13) or aeration means (17).

21. A plant according to claim 20, **characterized in that** the vessel (11) of the second reactor (10) is partly or completely produced from a transparent material and the lighting means (13) comprises a source of natural light (sunlight) or artificial light located outside said vessel.

22. A plant according to claim 20, **characterized in that** the lighting means (13) for the vessel (11) of the second reactor (10) comprises an artificial light source located inside said vessel.

23. A plant according to any one of claims 16 to 22, **characterized in that** the means (14) for recovering edible biomass comprises means (15) for evacuating said biomass from the second reactor vessel and phase separator means (16) for separating the biomass from the liquid effluent in which it is immersed.

24. A plant according to any one of claims 16 to 23, **characterized in that** the edible biomass packaging means (18) comprises draining means and/or sterilising means and/or dehydrating means.

25. A plant according to any one of claims 16 to 24, **characterized in that** it comprises a composting unit (20) for exploiting, in the form of compost, the residual solid material from the waste decomposition.

26. Application of the open circuit organic waste treatment method according to any one of claims 1 to 15 to recycling animal waste.

27. Application of the open circuit organic waste treatment method accordiçng to any one of claims 1 to 15 to recycling sludge from water purification.

28. Application of the open circuit organic waste treatment method according to any one of claims 1 to 15 to the manufacture of animal feeds.

## Patentansprüche

1. Verfahren zur Behandlung von organischem Abfall in einem offenen Kreislauf **dadurch gekennzeichnet, daß** es nachfolgende Schritte umfaßt:
a) die Sammlung des Abfalls,
b) die Einleitung des Abfalls ohne vorherige Sterilisation in einen ersten Reaktor,
c) den Abbau des Abfalls im besagten ersten Reaktor durch anaerobe mesophile oder thermophile Bakterien,
d) die Sammlung abfließender Flüssigkeit, die sich aus dem besagten Abbau ergibt und ihre Überführung in einen zweiten Reaktor, der heterotrophe oder photoheterotrophe Bakterien enthält,
e) die Erzeugung einer eßbaren Biomasse durch die heterotrophen oder photoheterotrophen Bakterien, die aus den besagten Bakterien besteht,
f) die Sammlung der so produzierten Biomasse und ihre Aufbereitung.

2. Verfahren nach Anspruch 1, **dadurch gekennzeichnet, daß** die anaeroben mesophilen oder thermophilen Bakterien, die den Abbau des Abfalls sicher stellen, eine Mischung von proteolytischen Bakterien, saccharolytischen Bakterien und cellulolytischen Bakterien sind.

3. Verfahren nach Anspruch 1 oder nach Anspruch 2, **dadurch gekennzeichnet, daß** die anaeroben mesophilen oder thermophilen Bakterien, die den Abbau des Abfalls sicher stellen. Bakterien sind, die im Magen der Tiere vorkommen.

4. Verfahren nach einem der vorhergehenden Ansprüche, **dadurch gekennzeichnet, daß** der Abbau des Abfalls durch die anaeroben thermophilen Bakterien bei einer Temperatur zwischen 45 und 80 °C und vorzugsweise zwischen 55 und 70°C durchgeführt wird.

5. Verfahren nach einem der vorhergehenden Ansprüche, **dadurch gekennzeichnet, daß** der Abbau des Abfalls innerhalb einer ausreichenden Zeit durchgeführt wird, um die Abbauraten der Proteine, Polysaccharide und Lipide, die im Abfall vorliegen, zu mehr als 80%, zu erreichen.

6. Verfahren nach einem der vorhergehenden Ansprüche, **dadurch gekennzeichnet, daß** die Sammlung der ausströmenden Ausfluß, die aus dem Abbau des Abfalls resultiert, einen Abtrennungsschritt der flüssigen und festen Phase umfaßt.

7. Verfahren nach Anspruch 6, **dadurch gekennzeichnet, daß** die Trennung der flüssigen und festen Phase der ausströmenden Ausfluß durch eine Filtration oder eine Siebung durchgeführt wird.

8. Verfahren nach einem der vorhergehenden Ansprüche, **dadurch gekennzeichnet, daß** die heterotrophen oder photoheterotrophen Bakterien, die die Produktion der eßbaren Biomasse sicher stellen, der Familie der *Rhodospirillaceae* angehören.

9. Verfahren nach Anspruch 8, **dadurch gekennzeichnet, daß** die heterotrophen oder photoheterotrophen Bakterien, die die Produktion der verwertbaren Biomasse sicher stellen, der Gattung der *Rhodobacter* und/oder der Gattung der *Rhodospirillum* angehören.

10. Verfahren nach einem der vorhergehenden Ansprüche, **dadurch gekennzeichnet, daß** die Produktion der eßbaren Biomasse heterotroph oder aerobiotisch durchgeführt wird.

11. Verfahren nach einem der vorhergehenden Ansprüche, **dadurch gekennzeichnet, daß** die Sammlung der eßbaren Biomasse einen Abtrennungsschritt der Biomasse von der abfließenden Ausfluß, in der sie sich befindet, umfaßt.

12. Verfahren nach Anspruch 11, **dadurch gekennzeichnet, daß** die Abtrennung der Biomasse von der abfließenden Ausfluß durch Flotation bewirkt wird.

13. Verfahren nach einem der vorhergehenden Ansprüche, **dadurch gekennzeichnet, daß** es die Entnahme verbrennbarer Gasprodukte, die während des Abbaus des Abfalls durch die anaeroben mesophilen oder thermophilen Bakterien, hergestellt sind, für ihre Verwertung zur Verbrennung umfaßt.

14. Verfahren nach Anspruch 13, **dadurch gekennzeichnet, daß** die Verbrennung der Gase verwendet wird, um den ersten Reaktor zu erhitzen.

15. Verfahren nach einem der vorhergehenden Ansprüche, **dadurch gekennzeichnet, daß** es die Kompostierung des zurückbleibenden festen Materials aus dem Abbau des Abfalls für ihre Verwertung in Form von Kompost umfaßt.

16. Einrichtung zur Durchführung eines Verfahrens zur Behandlung von organischem Abfall in einem offenen Kreislauf nach einem der Ansprüche 1 bis 11, **dadurch gekennzeichnet, daß** sie umfaßt:
a) einen ersten Reaktor (1) für den Abbau des Abfalls durch anaerobe mesophile oder thermophile Bakterien, wobei der genannte Reaktor eine Einfassung (2) umfaßt, die mit Vorrichtungen (3) für die Zuführung des Abfalls und mit Vorrichtungen (7) für die Sammlung der ausströmenden Ausfluß, die aus dem Abbau resultiert, ausgerüstet ist,
b) einen zweiten Reaktor (10) für die Produktion von eßbarer Biomasse durch die heterotrophen oder photoheterotrophen Bakterien, die aus den genannten Bakterien besteht, wobei der genannte zweite Reaktor (10) eine Einfassung (11) umfaßt, die mit Vorrichtungen (14) für die Sammlung der produzierten Biomasse ausgerüstet ist,
c) Vorrichtungen (12), um den resultierenden Ausfluß aus dem Abbau des Abfalls aus dem ersten Reaktor in den zweiten Reaktor zu überführen, und
d) Vorrichtungen (18) für die Aufbereitung der Biomasse.

17. Einrichtung nach Anspruch 16, **dadurch gekennzeichnet, daß** die Einfassung (2) des ersten Reaktors (1) mit Vorrichtungen (6) zum Erwärmen und/oder Vorrichtungen (4) für die Entnahme des während des Abbaus des Abfalls befreiten Gases in der genannten Einfassung ausgerüstet ist,.

18. Einrichtung nach Anspruch 16 oder Anspruch 17, **dadurch gekennzeichnet, daß** die Einfassung (2) des ersten Reaktors (1) mit Vorrichtungen zum Erhitzen und Vorrichtungen (4) zur Entnahme des während des Abbaus des Abfalls befreiten Gases ausgerüstet ist, und daß die Vorrichtungen zum Erwärmen ein Heizsystem umfassen, das durch die Vorrichtungen zur Gasentnahme der besagten Einfassung direkt oder durch den Zwischenträger einer Einrichtung (5) für die Gaslagerhaltung gespeist wird.

19. Einrichtung nach einem der Ansprüche 16 bis 18, **dadurch gekennzeichnet, daß** die Vorrichtungen (7) zur Sammlung der ausfließenden Flüssigkeit, die aus dem Abbau des Abfalls resultiert, die Vorrichtungen (8) für die Entleerung des Ausflusses aus der Einfassung des ersten Reaktors und die Vorrichtungen (9) zur Phasentrennung der flüssigen und festen Phasen, die sie darstellen, umfassen.

20. Einrichtung nach einem der Ansprüche 16 bis 20, **dadurch gekennzeichnet, daß** die Einfassung (11) des zweiten Reaktors (10) mit Vorrichtungen (13) zur Beleuchtung oder mit Vorrichtungen (17) zur Belüftung ausgerüstet ist.

21. Einrichtung nach Anspruch 20, **dadurch gekennzeichnet, daß** die Einfassung (11) des zweiten Reaktors (10) teilweise oder vollständig aus einem durchsichtigen Material besteht und die Vorrichtungen (13) zur Beleuchtung mit einer natürlichen (Sonnenlicht) oder künstlichen Lichtquelle sich außerhalb der besagten Einfassung befinden.

22. Einrichtung nach Anspruch 20, **dadurch gekennzeichnet, daß** die Vorrichtungen (13) zur Beleuchtung der Einfassung (11) des zweiten Reaktors (10) eine künstliche Lichtquelle, die sich innerhalb der besagten Einfassung befindet, umfassen.

23. Einrichtung nach einem der vorhergehenden Ansprüche 16 bis 22, **dadurch gekennzeichnet, daß** die Vorrichtungen (14) zur Sammlung der eßbaren Biomasse die Vorrichtungen (15) für die Entleerung der Biomasse von der Einfassung des zweiten Reaktors und die Vorrichtungen (16) zur Phasentrennung für die Abtrennung der ausströmenden Flüssigkeit, in welcher sie sich befindet, umfassen.

24. Einrichtung nach einem der vorhergehenden Ansprüche 16 bis 23, **dadurch gekennzeichnet, daß** die Vorrichtungen (18) der Aufbereitung der eßbaren Biomasse die Vorrichtungen für das Abtropfenlassen und/oder die Vorrichtungen für das Sterilisieren und/oder die Vorrichtungen für das Entwässern genannter Biomasse umfassen.

25. Einrichtung nach einem der vorhergehenden Ansprüche 16 bis 24, **dadurch gekennzeichnet, daß** sie eine Kompostierungseinheit (20) für die Verwertung des zurückbleibenden festen Materials aus dem Abbau des Abfalls in Form von Kompost umfaßt.

26. Anwendung des Verfahrens zur Aufbereitung von organischem Abfall im offenen Kreislauf nach einem der Ansprüche 1 bis 15 zur Rückführung des Abfalls tierischen Ursprungs.

27. Anwendung des Verfahrens zur Aufbereitung von organischem Abfall im offenen Kreislauf nach einem der Ansprüche 1 bis 15 zur Rückführung des durch die Reinigung des Wassers abgetrennten Schlamms.

28. Anwendung des Verfahrens zur Aufbereitung von organischem Abfall im offenen Kreislauf nach einem der Ansprüche 1 bis 15 für die Herstellung von Tierfutter.
